# EUROPEAN PATENT SPECIFICATION

(11) **EP 1 330 434 B1**
(45) Date of publication and mention of the grant of the patent: **12.12.2007**
(21) Application number: 01986676.3
(22) Date of filing: 09.10.2001
(51) Int. Cl.: C07C 319/02, C07C 321/26

(54) **PROCESS FOR THE PREPARATION OF THIOPHENOLS**
VERFAHREN ZUR HERSTELLUNG VON THIOPHENOLEN
PROCEDE RELATIF A L'ELABORATION DE THIOPHENOLS

(30) Priority: 11.10.2000 CH 200000
(43) Date of publication of application: 30.07.2003
(73) Proprietor: Syngenta Participations AG, 4058 Basel (CH)
(72) Inventor: SEIFERT, Gottfried, Breitenloh 5, 4333 Münchwilen (CH); URWYLER, Bernhard, Rothausweg, 4133 Schweizerhalle (CH)
(74) Representative: Arunasalam, Velautha-Cumaran
(86) International application number: PCT/EP2001/011653
(87) International publication number: WO 2002/030883

(56) References cited:
- DE-C- 433 103
- US-A- 5 428 002
- W. RUNDEL: "Notiz über die Darstellung tert.-butylierter Thiophenole, Diphenyldisulfide und Thianthrene" CHEM. BER., vol. 101, 1968, pages 2956-2962, XP001061836 cited in the application

## Description

The present invention relates to a new process for the preparation of thiophenols and to their use as intermediates in the preparation of herbicides of the isobenzofuranone type.

According to Methoden der organischen Chemie (Houben-Weyl), Volume 9, 12 (1955), thiophenol can be obtained, for example, by diazotising aniline and further reacting the resulting diazonium salt with potassium ethyl xanthogenate in a basic alcoholic medium.

According to DE 433103C, naphthalene derivatives can be obtained by reacting the corresponding diazonium salts of naphthalen-sulfonic acids in an aqueous alkaline solution of sulfur, followed by thermal cleavage of the sulfo groups in the generated intermediate bis-naphthalen-disulfide-disulfonic acids in diluted acids and reduction to the naphthalene-mercapto derivative.

Chem. Ber. 10, 2959 (1968) discloses that thiophenols are obtainable by reacting the phenyldiazonium salt with sodium polysulfide. According to The Chemistry of the Thiol Group, part 1, 220 (1974) and Chem. Ber. 120, 1161, disulfides can also be used instead of polysulfides, and disodium sulfide is described in Pestic. Sci. 1996, 47, 332 as a suitable reagent.

It has now been found, surprisingly, that the preparation of thiophenols can be significantly improved if, in the reaction of the corresponding phenyldiazonium salts, elemental sulfur is used instead of xanthogenates, polysulfides or sulfides.

The present invention accordingly relates to a process for the preparation of thiophenols of formula 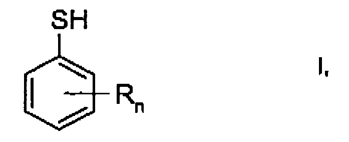 wherein
n is an integer from 1 to 5 and
R is hydrogen, alkyl, hydroxyalkyl, alkylamino, dialkylamino, alkenyl, alkynyl, alkoxy, alkylthio, phenyl, naphthyl, phenoxy, phenylthio, halogen, hydroxy, mercapto, carboxyl, sulfo, hydroxylamino or heterocyclyl,
by reacting phenyldiazonium salts of formula 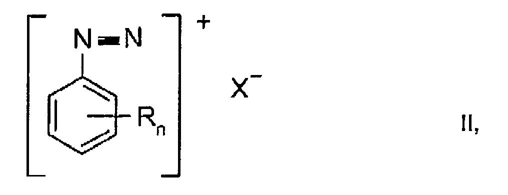
wherein n and R are as defined and X is halogen or hydrogen sulfate, with 1,2 to 5 mol of sulfur powder per mol of compound of formula II, at a temperature in the range of 20 to 100°C, in the presence of 2,5 to 5 mol per mol of compound of formula II of an aqueous base selected from alkali or alkaline earth metal hydroxide or ammonia, converting the excess of sulfur into water soluble thiosulfate by adding of sodium sulfite, sodium hydrogen sulfite or sulfur dioxide, acidifying the reaction solution, and isolating the compounds of formula I.

In the compounds of formula I, n is preferably an integer from 1 to 3. n is especially 2. Those compounds of formula I which have a carboxyl or sulfo group or a salt thereof have been found to be especially suitable and especially valuable. In particular, the compound of formula I which has a carboxyl group in the ortho position and a hydroxyalkyl group, especially a CH₃CH(OH) group, in the meta position (3 position) has proved to be especially advantageous. The phenyldiazonium salt of formula II corresponding to that compound is present in aqueous solution in a pH-dependent hydrolysis equilibrium with the corresponding closed lactone form, 7-mercapto-3-methyl-3H-isobenzofuran-1-one. That compound can be advantageously used in the preparation of herbicides of the isobenzofuranone type.

In general, compounds of formulae I and II that have, adjacent to one another, substituents capable of together forming a (fused-on) ring, for example a carboxyl group in the ortho position and a hydroxyalkyl group in the meta position, are present in aqueous solution in a pH-dependent equilibrium with the corresponding closed form, e.g. the lactone form, in particular the 5-membered ring lactone form, for example in 7-mercapto-3-methyl-3H-isobenzofuran-1-one, being especially readily formed in acid solution. As a rule, the tendency towards ring formation decreases with increasing ring size. 6- and 7-membered rings generally form less readily than the 5-membered rings.

The process according to the invention therefore also encompasses the preparation of those closed forms of the compounds of formula I wherein two substituents R have formed a fused-on ring.

The alkyl radicals appearing in the definitions of R preferably contain from 1 to 4 carbon atoms and are, for example, methyl, ethyl, propyl and butyl and branched isomers thereof. Preferred alkoxy, alkylthio and hydroxyalkyl radicals are derived from the mentioned alkyl radicals. Alkenyl and alkynyl radicals R preferably have from 2 to 4 carbon atoms and are, for example, ethenyl, propenyl, ethynyl, propynyl and propenyl and branched isomers thereof, and butenyl and butynyl and branched and di-unsaturated isomers thereof. The terms hydroxy (-OH), mercapto (-SH) and sulfo (-SO₃H) and carboxyl (-CO₂H) also include in each case the salt form thereof, for example alkali metal, alkaline earth metal and ammonium salts. Heterocyclyl is understood to mean preferably from 4- to 8-membered, saturated or unsaturated rings that contain at least one hetero atom selected from nitrogen, sulfur and oxygen. Examples thereof are pyridyl, furanyl, thiofuranyl, oxetanyl, thiazinyl, morpholinyl, piperazinyl, pyridazinyl, pyrazinyl, thiopyranyl, pyrazolyl, pyrimidinyl, triazinyl, isofuranyl, pyranyl, piperidyl, picolinyl, thiadiazolinyl, thietanyl, triazolyl, oxazolanyl, thiolanyl, azepinyl, thiazolyl, isothiazolyl, imidazolyl or pyrrolyl.

Preference is given to the use of sulfur in the form of a powder, especially from 1.5 to 3 mol, of sulfur per mol of phenyldiazonium salt.

The diazonium salts are prepared in known manner by metering a sodium nitrite solution into the acid solution of the corresponding amine in water at temperatures of about from -5°C to +5°C.

It is especially advantageous to proceed at a temperature in a range of from 30 to 80°C.

Preference is given to the use of from 1.5 to 3 mol, of an aqueous solution of an alkali or alkaline earth metal hydroxide or ammonia per mol of diazonium compound. If the phenyldiazonium salt of formula II already contains acid groups as substituents, preferably an additional mole of base is required for each acid group.

The process according to the invention has the major advantage that it can be carried out on a large industrial scale. The procedure is generally that the sulfur is introduced into the aqueous base at elevated temperature and the diazonium salt of formula II is metered in.

The process according to the invention can be carried out either continuously or intermittently (discontinuously, batch-wise), with preference being given to intermittent operation. Both the intermittent and the continuous reaction procedures are carried out preferably in a stirred vessel or a stirred vessel cascade.

It has been found to be advantageous, for isolation of the thiols, to convert the excess of sulfur into sodium thiosulfate by adding sodium sulfite, sodium hydrogen sulfite or sulfur dioxide. The reaction solution is then acidified, whereupon the thiol separates out and can be isolated from the aqueous salt solution. For further purification the phenylthiol can be distilled.

The yields of isolated thiol are generally from 80 to 100 %. The chemical yield in the reaction mixture is usually more than 95 %.

The process according to the invention has the following advantages over the processes of the prior art:
- it can be performed on a large industrial scale
- the reaction procedure is simple, especially compared to the disulfide variant, where disulfide solution and diazo solution have to be metered into the aqueous base simultaneously in order to suppress the exchange of the diazo group for hydrogen
- there is no formation of toxic subsidiary products as are formed especially in the case of the xanthogenate method, in which COS is formed in molar amounts during working-up
- compared to the disulfide and polysulfide variant, the reaction proceeds very selectively
- it results in products in yields of up to 98 %
- it can be performed in a multi-purpose apparatus.

The thiols of formula I prepared according to the invention are used, in particular, as intermediates in the preparation of herbicides of the isobenzofuranone type, which are described, for example, in US-A-5 332 717 and US-A-5 428 002.

The Examples that follow illustrate the invention further.

### Example 1. Preparation of 7-mercapto-3-methyl-3H-isobenzofuran-1-one

In a first reactor, 203 g of 2-amino-6-(1-hydroxyethyl)-benzoic acid (sodium salt) in the form of a 50 % aqueous solution (1.0 mol) and 181 g of a 40 % aqueous sodium nitrite solution (1.05 mol) are introduced simultaneously into 428 g of hydrochloric acid (32 %). After the reaction is complete, excess nitrite is destroyed by the addition of sulfamic acid.

In a second reactor, 800 g of sodium hydroxide in the form of a 30 % solution and 71 g of sulfur powder (2.20 mol) are heated at 60°C and stirred for 60 minutes. The diazonium salt solution prepared in the first reactor is added to the resulting mixture, the corresponding thiol being formed with evolution of nitrogen.

For working up, 300 g of toluene and 195 g of sodium hydrogen sulfite (0.75 mol) in the form of a 40 % aqueous solution are added to the reaction mixture. At a temperature of 80°C, the pH is adjusted to 8 using acetic acid, as a result of which the phthalide ring closes and excess sulfur is converted into thiosulfate. At 30°C, as a result of further lowering the pH to 6, the thiol is liberated and taken up into the toluene phase. The toluene phase is separated off and 5 g of triphenylphosphine and 150 g of water are added thereto. Heating to about 30°C is carried out and the pH is adjusted to 11, whereupon the phenylthiol in the form of its sodium salt is taken up into the aqueous phase, from which 7-mercapto-3-methyl-3H-isobenzofuran-1-one in the form of its sodium salt can be isolated, in a yield of 85 % (based on 2-amino-6-(1-hydroxyethyl)-benzoic acid) in the form of an aqueous solution.

## Claims

1. A process for the preparation of a thiophenol of formula 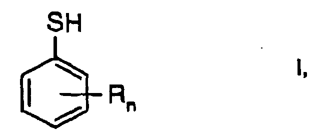 wherein
n is an integer from 1 to 5 and
R is hydrogen, alkyl, hydroxyalkyl, alkylamino, dialkylamino, alkenyl, alkynyl, alkoxy, alkylthio, phenyl, naphthyl, phenoxy, phenylthio, halogen, hydroxy, mercapto, carboxyl, sulfo or heterocyclyl,
by reacting a phenyldiazonium salt of formula 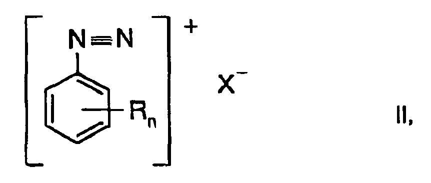
wherein n and R are as defined and X is halogen or hydrogen sulfate, with 1,2 to 5 mol of sulfur powder per mol of compound of formula II, at a temperature in the range of 20 to 100°C, in the presence of 2.5 to 5 mol per mol of compound of formula II of an aqueous base selected from alkali or alkaline earth metal hydroxide or ammonia,
converting the excess of sulfur into water soluble thiosulfate by adding of sodium sulfite, sodium hydrogene sulfite or sulfur dioxide,
acidifying the reaction solution, and
isolating the compound of formula I.

2. The process according to claim 1 wherein 1.5 to 3 mol of sulfur is used per mol of compound of formula II.

3. The process according to either claim 1 or 2 wherein the temperature range of from 30 to 80°C.

4. The process according to any one of claims 1 to 3 wherein 1.5 to 3 mol of aqueous base is used per mol of compound of formula II

## Patentansprüche

1. Verfahren zur Herstellung eines Thiophenols der Formel (I) : 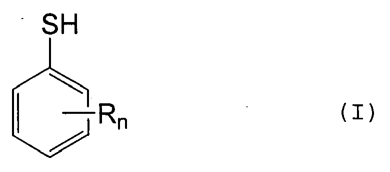 worin
n eine ganze Zahl von 1 bis 5 ist und
R Wasserstoff, Alkyl, Hydroxyalkyl, Alkylamino, Dialkylamino, Alkenyl, Alkinyl, Alkoxy, Alkylthio, Phenyl, Naphthyl, Phenoxy, Phenylthio, Halogen, Hydroxy, Mercapto, Carboxyl, Sulfo oder Heterocyclyl ist,
durch Umsetzen eines Phenyldiazoniumsalzes der Formel (II): 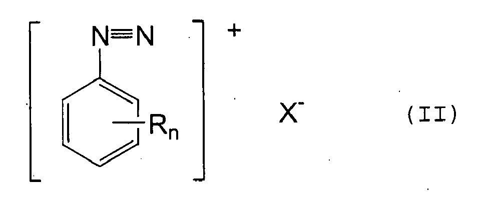
worin n und R wie oben definiert sind und X Halogen oder Hydrogensulfat ist,
mit 1,2 bis 5 mol Schwefelpulver pro Mol der Verbindung der Formel (II) bei einer Temperatur im Bereich von 20 bis 100°C, in Gegenwart von 2,5 bis 5 mol einer wäßrigen Base ausgewählt aus Alkali- oder Alkalierdmetallhydroxid oder Ammonia pro Mol der Verbindung der Formel (II),
Umwandeln des Überschusses an Schwefel in wasserlösliches Thiosulfat durch Zugeben von Natriumsulfit, Natriumhydrogensulfit oder Schwefeldioxid,
Ansäuern der Reaktionslösung und
Isolieren der Verbindung der Formel (I).

2. Verfahren gemäß Anspruch 1, worin 1,5 bis 3 mol Schwefel pro Mol der Verbindung der Formel (II) verwendet werden.

3. Verfahren entweder gemäß Anspruch 1 oder 2, worin der Temperaturbereich von 30 bis 80°C liegt.

4. Verfahren gemäß irgendeinem der Ansprüche 1 bis 3, worin 1,5 bis 3 mol einer wäßrigen Bas pro Mol der Verbindung der Formel (II) verwendet werden.

## Revendications

1. Procédé de préparation d'un thiophénol de formule 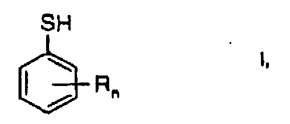 dans laquelle
n représente un nombre entier de 1 à 5 et
R représente un atome d'hydrogène, un groupe alkyle, hydroxyalkyle, alkylamino, dialkylamino, alcényle, alcynyle, alcoxy, alkylthio, phényle, naphtyle, phénoxy, phénylthio, un atome d'halogène, un groupe hydroxy, mercapto, carboxyle, sulfo ou hétérocyclyle,
en faisant réagir un sel de phényldiazonium de formule 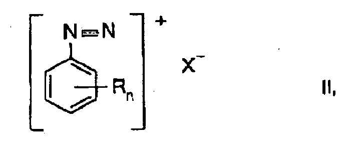
dans laquelle n et R sont tels que définis et X représente un atome d'halogène ou un groupe hydrogénosulfate, avec 1,2 à 5 mol de soufre en poudre par mol de composé de formule II, à une température dans la plage de 20 à 100°C, en présence de 2,5 à 5 mol par mol de composé de formule II d'une base aqueuse choisi parmi un hydroxyde de métal alcalin ou alcalino-terreux ou l'ammoniaque,
en convertissant l'excès de soufre en thiosulfate hydrosoluble par l'addition de sulfite de sodium, d'hydrogénosulfite de sodium ou de dioxyde de soufre,
en acidifiant le solution réactionnelle, et
en isolant le composé de formule I.

2. Procédé selon la revendication 1, dans lequel 1,5 à 3 mol de soufre sont utilisées par mol de composé de formule II.

3. Procédé selon l'une ou l'autre de la revendication 1 ou 2, dans lequel la température se situe dans la plage de 30 à 80°C.

4. Procédé selon l'une quelconque des revendications 1 à 3, dans lequel 1,5 à 3 mol de base aqueuse sont utilisées par mol de composé de formule II.
